# EUROPEAN PATENT APPLICATION

(11) **EP 4 545 630 A1**
(43) Date of publication of application: **30.04.2025**
(21) Application number: 24747382.0
(22) Date of filing: 26.01.2024
(51) Int. Cl.: C12N 5/0775, A61K 35/28, A61P 9/10, A61P 17/02, A61P 25/16, A61P 25/28, C12M 3/00

(54) **CELL PROLIFERATION CONTROL METHOD IN AUTOMATIC CULTURE DEVICE**

(30) Priority: 27.01.2023 JP 2023011235
(71) Applicant: Rainbow Inc., Sapporo-shi, Hokkaido, 001-0021 (JP)
(72) Inventor: KAWABORI, Masahito, Sapporo-shi, Hokkaido 060-0808 (JP)
(74) Representative: Vossius & Partner Patentanwälte Rechtsanwälte mbB
(86) International application number: PCT/JP2024/002529
(87) International publication number: WO 2024/158058

(57) **Abstract**

The present disclosure provides a method for causing stem cells to proliferate while suppressing proliferation. **In** one aspect, the present disclosure provides a method for causing stem cells to proliferate while suppressing proliferation, the method including a step of culturing the stem cells under a hibernation condition of the stem cells. **In** another aspect, the present disclosure provides a method for producing a predetermined amount of stem cells in a timely manner, the method including: a step (a) of culturing the stem cells under a hibernation condition of the stem cells; and a step (b) of culturing the stem cells to a predetermined amount of the stem cells at about 36°C to 38°C.

## Description

### Technical Field

The present disclosure relates to a technique for controlling a proliferation rate of stem cells.

### Background Art

Regenerative medicine has attracted attention as a novel treatment method for central nervous diseases (cerebral infarction, cerebral hemorrhage, head injury, Parkinson's disease, and the like), and development of a plurality of cell medicines has been advanced. The present inventors have previously developed a treatment by direct transplantation in which cells are directly delivered into the brain, and a high treatment result has been obtained (Non Patent Literature 1).

### Citation List

### Non Patent Literature

Non Patent Literature 1: Shichinohe, Kawabori et al. Research on advanced intervention using novel bone marrow stem cell (RAINBOW): a study protocol for a phase I, open-label, uncontrolled, dose-response trial of autologous bone marrow stromal cell transplantation in patients with acute ischemic stroke BMC Neurol. 2017 Sep 8; 17 (1): 179

### Summary of Invention

### Solution to Problem

The present inventors have found a hibernation condition for suppressing proliferation of stem cells, and found that proliferation of stem cells can be controlled by the hibernation condition.

The present invention provides, for example, the following items.

### (Item 1)

A method for causing stem cells to proliferate while suppressing proliferation, the method including a step of culturing the stem cells under a hibernation condition of the stem cells.

### (Item 2)

A method for causing stem cells to proliferate while suppressing proliferation, the method including a step of culturing the stem cells at a temperature of about 20°C to about 34°C.

### (Item 3)

The method according to Item 1, in which the hibernation condition is a temperature of about 25°C to about 34°C.

### (Item 4)

The method according to Item 1, in which the hibernation condition is a temperature of about 28°C to about 32°C.

### (Item 5)

The method according to Item 1, in which the hibernation condition is a temperature of about 30°C to about 32°C.

### (Item 6)

The method according to any one of Items 1 to 5, in which the method is performed without passaging.

### (Item 7)

The method according to any one of Items 1 to 6, in which the method is performed in an automatic culture device.

### (Item 8)

The method according to any one of Items 1 to 7, in which the stem cells are mesenchymal stem cells.

### (Item 9)

The method according to Item 8, in which the mesenchymal stem cells are mesenchymal stem cells stem cells derived from bone marrow, adipose tissue, placental tissue, synovial tissue, umbilical cord tissue (for example, cord blood), dental pulp, or amniotic membrane, or mesenchymal stem cells differentiated from ES cells or iPS cells.

### (Item 10)

The method according to Item 9, in which the mesenchymal stem cells are bone marrow-derived mesenchymal stem cells.

### (Item 11)

The method according to any one of Items 1 to 10, in which the cells are cells used in any of cell infusion therapy and drug discovery research.

### (Item 12)

A method for producing a predetermined amount of stem cells in a timely manner, the method including: a step (a) of culturing the stem cells under a hibernation condition of the stem cells; and a step (b) of culturing the stem cells to a predetermined amount of the stem cells at about 36°C to 38°C.

### (Item 13)

The method according to Item 12, in which the hibernation condition is a temperature of about 20°C to about 34°C.

### (Item 14)

The method according to Item 12, in which the hibernation condition is from about 28°C to about 32°C.

### (Item 15)

The method according to Item 12, in which the hibernation condition is from about 30°C to about 32°C.

### (Item 16)

A method for producing a predetermined amount of stem cells in a timely manner, the method including: a step (a) of culturing the stem cells at a temperature of the stem cells of about 20°C to about 34°C; and a step (b) of culturing the stem cells to a predetermined amount of the stem cells at about 36°C to 38°C.

### (Item 17)

The method according to any one of Items 12 to 16, in which the method is performed without passaging.

### (Item 18)

The method according to any one of Items 12 to 17, in which the method is performed in an automatic culture device.

### (Item 19)

The method according to any one of Items 12 to 18, in which the stem cells are mesenchymal stem cells.

### (Item 20)

The method according to Item 19, in which the mesenchymal stem cells are mesenchymal stem cells stem cells derived from bone marrow, adipose tissue, placental tissue, synovial tissue, umbilical cord tissue (for example, cord blood), dental pulp, or amniotic membrane, or mesenchymal stem cells differentiated from ES cells or iPS cells.

### (Item 21)

The method according to Item 19, in which the mesenchymal stem cells are bone marrow-derived mesenchymal stem cells.

### (Item 22)

The method according to any one of Items 12 to 21, in which the cells are cells used in cell infusion therapy and drug discovery research.

### (Item 23)

Cells obtained by the method according to any one of Items 1 to 22.

### (Item 24)

A pharmaceutical composition containing cells obtained by the method according to any one of Items 1 to 22.

### (Item 25)

The pharmaceutical composition according to Item 24, in which the pharmaceutical composition is used for treating or preventing severe burns, spinal cord injury, head injury, cerebral infarction, cerebral hemorrhage, Parkinson's disease, or dementia.

### (Item 26)

A system for causing stem cells to proliferate while suppressing proliferation, the system including:
a culture vessel for culturing stem cells; and
a temperature controller that controls a temperature of the culture vessel,
in which the temperature controller controls the temperature to culture the stem cells under a hibernation condition of the stem cells.

### (Item 27)

A system for producing a predetermined amount of stem cells in a timely manner, the system including:
a culture vessel for culturing stem cells;
a temperature controller that controls a temperature of the culture vessel; and
a cell amount measuring means for detecting whether or not the amount of cells is a predetermined amount,
in which the temperature controller controls the temperature to culture the stem cells under a hibernation condition and a normal culture condition of the stem cells.

### (Item 28)

A system for causing stem cells to proliferate while suppressing proliferation, the system including:
a culture vessel for culturing stem cells; and
a temperature controller that controls a temperature of the culture vessel,
in which the temperature controller controls the temperature to culture the stem cells at about 20°C to 34°C.

### (Item 29)

A system for producing a predetermined amount of stem cells in a timely manner, the system including:
a culture vessel for culturing stem cells;
a temperature controller that controls a temperature of the culture vessel; and
a cell amount measuring means for detecting whether or not the amount of cells is a predetermined amount,
in which the temperature controller controls the temperature to culture the stem cells at about 20°C to 34°C and about 36°C to 38°C.

In the present disclosure, it is intended that the one or a plurality of features may be provided in further combination in addition to the specified combination. Further embodiments and advantages of the present disclosure will be appreciated by those skilled in the art upon reading and understanding the following detailed description, if necessary.

### Advantageous Effects of Invention

According to the present disclosure, proliferation of stem cells can be controlled, and an appropriate amount of stem cells can be provided in a timely manner.

### Brief Description of Drawings

FIG. 1 shows a graph of a transition of the number of living cells when cells are cultured at 37°C for 2 days, at 30°C for 3 days, and then at 37°C for 3 days.
FIG. 2 shows a graph of a transition of the number of living cells when cells are cultured at 37°C for 2 days, at 30°C, 32°C, or 34°C for 3 days, and then at 37°C for 3 days.
FIG. 3 shows a graph in which an average of results in FIG. 2 is put together into one graph.
FIG. 4 shows a graph of a transition of the number of living cells when cells are cultured at 37°C for 2 days, at 32°C for 3 days, and then at 37°C for 8 days.
FIG. 5 shows a graph in which an average of results in FIG. 4 is put together into one graph.
FIG. 6 shows a graph of an HGF ratio per cell after a temperature change. The group with no temperature change was defined as 1.
FIG. 7 shows a comparison between the final number of cells in a case of culturing using an automatic culture device and the number of cells predicted from a lactic acid value.
FIG. 8 shows a graph of a transition of the number of cells in culture using an automatic culture device.
FIG. 9 shows a graph of a transition of the number of cells when cells are cultured at 37°C for 2 days, at 20°C for 3 days, and then at 37°C for 3 days.
FIG. 10 shows a graph in which an average of results in FIG. 9 is put together into one graph.
FIG. 11 shows a graph of a transition of the number of cells when cells are cultured at 37°C for 2 days, at 25°C for 3 days, and then at 37°C for 3 days.
FIG. 12 shows a graph in which an average of results in FIGS. 9 and 11 is put together into one graph.

### Detailed Description of Embodiments

Hereinafter, the present disclosure will be described. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used in the present specification are to be understood as being used in the sense commonly used in the art unless otherwise stated. Therefore, unless defined otherwise, all technical and scientific terms used in the present specification have the same meaning as commonly understood by those skilled in the art to which the present disclosure pertains. In the case of conflict, the present specification (including definitions) will control.

### (Definitions)

In the present specification, "about" means ±10% of a subsequent value.

In the present specification, "stem cell" refers to a cell having self-replication ability to divide to produce the same cell as itself and differentiation ability to differentiate into a specific cell. The stem cell is not particularly limited as long as it is a cell having self-replication ability and differentiation ability, and may be a pluripotent stem cell or a somatic stem cell. The pluripotent stem cell is a cell having self-replication ability and differentiation ability to differentiate into any of ectoderm, mesoderm, and endoderm. Examples of the pluripotent stem cells include embryonic stem cells (ES cells), induced pluripotent stem cells (iPS cells), embryonic germ cells (EG cells), embryonic cancer cells (EC cells), multipotent adult progenitor cells (MAP cells), adult pluripotent stem cells (APS cells), and Muse cells. Examples of the somatic stem cells include mesenchymal stem cells, hematopoietic stem cells, and neural stem cells.

In the present specification, the term "mesenchymal stem cell" is also referred to as a mesenchymal stromal cell, and refers to a stem cell having differentiation ability to differentiate into a cell belonging to a mesenchymal system, such as an osteoblast, an adipocyte, a myocyte, or a chondrocyte. The mesenchymal stem cells can include mesenchymal stem cells stem cells derived from bone marrow, adipose tissue, placental tissue, synovial tissue, umbilical cord tissue (for example, cord blood), dental pulp, or amniotic membrane, or mesenchymal stem cells differentiated from ES cells or iPS cells.

In the present specification, "proliferation" (of stem cells) refers to dividing to produce the same cell as itself and increasing the number of cells.

In the present specification, "suppression" of "proliferation" refers to stopping the proliferation of cells or reducing the culture rate as compared to culture under normal culture conditions without completely stopping the proliferation of cells.

In the present specification, "hibernation condition" refers to a condition in which the cell cycle stops and proliferation is suppressed. When the hibernation condition is released, cell proliferation may resume. Such a condition includes, but is not limited to, a temperature, a medium component, a pH, an osmotic pressure, an oxygen or carbon dioxide concentration, and the like.

In the present specification, "normal culture condition" refers to a condition suitable for causing specific cells to proliferate. Such a condition includes, but is not limited to, a temperature, a medium component, a pH, an osmotic pressure, an oxygen or carbon dioxide concentration, and the like. The normal culture condition in the culture of stem cells may typically be a culture medium containing about 36 to about 38°C (preferably about 37°C), a 5% CO² atmosphere, a pH of 6 to 8, and any proliferation factor.

In the present specification, "without passaging" refers to not transferring cells proliferated in a culture vessel to a new vessel to continue maintaining the culture, that is, continuing the culture in the same vessel.

In the present specification, "automatic culture device" refers to a device that automatically performs operations from cell input to recovery of cultured cells without manual work. Specific examples of the automatic culture device include, but are not limited to, Quantum (TERUMO BCT), CliniMACS Prodigy (Miltenyi Biotec), and CELLAFORTE (Nipro). Other automatic culture devices are disclosed in, for example, Mishiro SHIMIZU et al., Medical Instrumentation Vol. 88, No. 4 (2018) (17).

In the present specification, "cell infusion therapy" refers to a therapeutic method of infusing cells for a treatment of a disease.

In the present specification, "drug discovery research" refers to exploratory research, development research, and clinical research in the discovery of drug.

In the present specification, "cells used in cell infusion therapy" refer to cells infused in cell infusion therapy. Specifically, mesenchymal stem cells (regardless of whether they are derived from bone marrow, amniotic membrane, dental pulp, or the like), embryonic stem cells (ES cells), iPS cells, and the like are included.

In the present specification, "cells used for drug discovery research" refer to cells used in a process of exploratory research, development research, or clinical research, or cells as a candidate actually used as a drug. Specifically, such cells include ES cells and iPS cells.

In the present specification, "producing a predetermined amount of stem cells in a timely manner" refers to providing stem cells by culturing so as to achieve the cell number or cell density of the stem cells set according to the purpose at the culture end time point set according to the purpose.

The predetermined amount can be appropriately determined according to the purpose. Illustratively, about 50 million to 100 million cells may be sufficient to infuse 40 million cells in cell infusion therapy.

A desired cell density is preferably a cell density reaching a confluent cell density or less, and can be appropriately set.

The culture end time point set according to the purpose refers to a culture end time point set so that a predetermined amount of cells can be provided until the purpose (for example, cell infusion therapy) is performed.

In the present specification, "drug" or "pharmaceutical composition" refers to a drug used for preventing or treating a disease or a composition containing the drug.

In the present specification, "cell preparation" refers to a preparation containing cells, which is in a form suitable for use or in a form of preparation in use. "Preparation in use" refers to preparing a formulation suitable for use by adding a drug or diluting with a solvent just immediately to administration.

In the present specification, "prevention" (of severe burns, spinal cord injury, head injury, cerebral infarction, cerebral hemorrhage, Parkinson's disease, or dementia) refers to delaying the onset of these diseases, not causing these diseases to develop or reducing symptoms at the onset of these diseases.

In the present specification, "treatment" (of severe burns, spinal cord injury, head injury, cerebral infarction, cerebral hemorrhage, Parkinson's disease, or dementia) refers to alleviating or eliminating these diseases or symptoms of these diseases.

### (Preferred embodiments)

Although preferred embodiments are described below, it is to be understood that these embodiments are illustrative of the present disclosure and that the scope of the present invention is not limited to these preferred embodiments. It should be understood that those skilled in the art can also easily implement modifications, changes, and the like within the scope of the present disclosure with reference to the following preferred examples. For these embodiments, those skilled in the art may appropriately combine any embodiments.

### (Application of hibernation condition)

Currently, regenerative medicine products using stem cells are beginning to be approved, but there is a problem of high culture costs (labor cost of workers by manual work and culture room cleanliness maintenance cost). The solution is to automate production using an automatic culture device, and in particular, an automatic culture device that does not require cell passaging is expected to significantly reduce costs because it can perform the entire process from inputting the stem cell source to recovering the final product without manual work and can ensure cleanliness by using a disposable kit. However, as a problem of the automatic culture device that does not require cell passaging, it is difficult to manage proliferation of stem cells. In normal flask culture, the number of cells can be controlled by controlling the number of cells at the time of cell passaging, but the number of cells cannot be controlled using an automatic culture device that does not require passaging. That is, after the application, subsequent proliferation control becomes difficult. In the first place, a proliferation rate of stem cells varies depending on the type of cells, the nature of the donor (age and the like), the type of culture solution, additives, and the like. When cells proliferate excessively, a culture solution for supplying nutrients is required more than expected, which is costly or insufficient with a prepared culture solution amount, and the culture needs to be interrupted or finished early. In a case of stem cells to be shipped without being frozen, the cells will not be delivered on the scheduled date for administration, and in a case of an autologous cell product, even when the amount of stem cells greatly exceeds the amount required for the administration, the amount cannot be administered, resulting in waste. A technique for keeping the required number of cells within a predetermined range is a significantly important technique in an automatic culture device that does not require passaging, but the method thereof has not been established. The present disclosure provides a solution to such problems.

A medium that can be applied in cell culture in the present disclosure is not particularly limited, and any medium can be applied. Specifically, the medium may be a liquid medium such as a basic medium for mammalian cells (for example, Dulbecco's Modified Eagle's Medium (DMEM), Dulbecco's Modified Eagle Medium: Nutrient Mixture F-12 (DMEM/F-12), Eagle's minimal essential medium (EMEM), Basal Medium Eagle (BME), Roswell Park Memorial Institute 1640 Medium (RPMI 1640 Medium), E8 base medium, Skeletal Muscle Cell Basal Medium (SkBM), MCDB 104, MCDB153, 199, or L15), a commercially available culture solution for maintaining stem cells, a basic medium for insect cells, a medium for yeast, or a medium for bacteria. The medium that can be applied to cell culture may contain various components that can be generally added, for example, antibiotics such as penicillin and streptomycin; vitamins or vitamin derivatives such as ascorbic acid and retinoic acid; sugar source such as glucose; amino acid; inorganic salts; serum and serum replacements; proteins such as transferrin; hormones such as insulin; proliferation factors; differentiation inhibitors; antioxidants such as 2-mercaptoethanol and dithiothreitol; metal ions such as calcium ions, magnesium ions, zinc ions, iron ions, and copper ions; and the like. In cell culture, a medium is altered by metabolites and the like secreted from cells. Therefore, at an appropriate time within the culture period, a medium replacement treatment for replacing the used medium in the cell culture vessel with a new medium may be performed.

A cell culture device that can be used in the present disclosure includes a cell supply unit, a medium supply unit, and if necessary, another component supply unit, and the like. In addition, the cell culture device may optionally include a cell culture vessel, a storage vessel, a division processing unit, a waste liquid collection vessel, and the like. The cell culture device stores cells supplied from the cell supply unit in a cell culture vessel together with a medium (culture solution) supplied from the medium supply unit, and cultures the cells in a state where the cells are, for example, suspended in the medium in the cell culture vessel. The device of the present disclosure may include a temperature sensing unit such as a thermometer that senses a temperature and a temperature control unit (such as a heating means and/or a cooling means) that controls a temperature. The heating means may be any heater or the like, and the cooling means may be any refrigerator or refrigerant or the like. The temperature control unit may realize heating and cooling by one device. The cell culture device of the present disclosure can include a control unit that controls these units. The control unit can be controlled by a program or the like described in the present disclosure.

The cell supply unit may include a cell storage unit that stores cells to be cultured by the cell culture device, and a pump that delivers the cells stored in the cell storage unit to a flow path configured to include a pipe. The cell supply unit includes an on-off valve provided on the downstream side of the pump of the pipe connecting the cell storage unit and the pipe. The cells stored in the cell storage unit may be delivered to the flow path by driving the pump and opening the on-off valve.

An inner wall surface of the cell culture vessel may be formed of a plastic material, and the inner wall surface of the cell culture vessel may be formed of, for example, metal, glass, ceramic, or the like. In this case, it is possible to form a non-flat structure by, for example, spraying, sandblasting, or etching. In addition, the entire cell culture vessel may be formed of metal, glass, ceramic, or the like. From the viewpoint of enhancing the effect of inhibiting cell adhesion, the inner wall surface of the cell culture vessel can also be formed of a plastic material. Alternatively, it is possible to use a plastic film as an inexpensive single-use cell culture bag by using a plastic film having a non-flat structure on a surface thereof as a base material of a cell culture vessel.

In one aspect, the present disclosure provides a method for causing stem cells to proliferate while suppressing proliferation, the method including a step of culturing the stem cells under a hibernation condition of the stem cells.

In another aspect, the present disclosure provides a method for producing a predetermined amount of stem cells in a timely manner, the method including: a step (a) of culturing the stem cells under a hibernation condition of the stem cells; and a step (b) of culturing the stem cells to a predetermined amount of the stem cells at about 36°C to 38°C.

The method of the present disclosure is particularly advantageous in culture without subculture, for example, culture in an automatic culture device without subculture. Furthermore, the method of the present disclosure is also advantageous in cell infusion therapy and drug discovery research that require specific amounts of stem cells at specific times.

In some embodiments, a period of time that the cells are subjected to the hibernation condition can be determined as appropriate, and the cells may be subjected to the hibernation condition for 1 to 10 days, for example, 1 day, 2 days, 3 days, 4 days, 5 days, 6 days, 7 days, 8 days, 9 days, or 10 days. Since the cells need to proliferate normally when rewarmed to normal temperature (for example, 37°C), when the period of the hibernation condition is too long, cell proliferation may not be controlled again; thus, the period of the hibernation condition is preferably up to 10 days, more preferably up to 5 days, and most preferably up to 3 days.

In some embodiments, the hibernation condition may be a temperature. In some embodiments, the hibernation condition is a condition in which cell proliferation is suppressed and cells proliferate normally when the hibernation condition is released, and can be, for example, a temperature of about 20°C to about 34°C, preferably about 25°C to about 34°C, more preferably about 28°C to about 32°C, and most preferably about 30°C to about 32°C.

With reference to Examples 1 to 7, the period and temperature of the hibernation condition, and the total culture period can be appropriately determined so as to achieve the cell number or cell density of stem cells set according to the purpose at the end of culture set according to the purpose.

In some embodiments, the stem cells can be mesenchymal stem cells. In some embodiments, the mesenchymal stem cells can be mesenchymal stem cells stem cells derived from bone marrow, adipose tissue, placental tissue, synovial tissue, umbilical cord tissue (for example, cord blood), dental pulp, or amniotic membrane, or mesenchymal stem cells differentiated from ES cells or iPS cells. In a preferred embodiment, the stem cells may be bone marrow-derived mesenchymal stem cells.

In still another aspect, the present disclosure provides cells obtained by the method and a pharmaceutical composition containing the cells. The cells and the pharmaceutical composition can be used for treating or preventing severe burns, spinal cord injury, head injury, cerebral infarction, cerebral hemorrhage, Parkinson's disease, or dementia.

### (Culture at specific temperature)

In another aspect, the present disclosure provides a method for causing stem cells to proliferate while suppressing proliferation, the method including a step of culturing the stem cells at about 20°C to about 34°C of the stem cells.

In another aspect, the present disclosure provides a method for producing a predetermined amount of stem cells in a timely manner, the method including a step (a) of culturing the stem cells at about 20°C to about 34°C of the stem cells; and a step (b) of culturing the stem cells to a predetermined amount of the stem cells at about 36°C to 38°C.

The method of the present disclosure is particularly advantageous in culture without subculture, for example, culture in an automatic culture device without subculture. Furthermore, the method of the present disclosure is also advantageous in cell infusion therapy and drug discovery research that require specific amounts of stem cells at specific times.

In some embodiments, a period of time that the cells are subjected to the hibernation condition can be determined as appropriate, and the cells may be subjected to the hibernation condition for 1 to 10 days, for example, 1 day, 2 days, 3 days, 4 days, 5 days, 6 days, 7 days, 8 days, 9 days, or 10 days. Since the cells need to proliferate normally when rewarmed to normal temperature (for example, 37°C), the period of the hibernation condition may be preferably up to 10 days, more preferably up to 5 days, and most preferably up to 3 days.

In some embodiments, the hibernation condition may be a temperature. In some embodiments, the hibernation condition is a condition in which cell proliferation is suppressed and cells proliferate normally when the hibernation condition is released, and can be, for example, a temperature of about 20°C to about 34°C, preferably about 25°C to about 34°C, more preferably about 28°C to about 32°C, and most preferably about 30°C to about 32°C.

With reference to Examples 1 to 7, the period and temperature of the hibernation condition, and the total culture period can be appropriately determined so as to achieve the cell number or cell density of stem cells set according to the purpose at the end of culture set according to the purpose.

In some embodiments, the stem cells can be mesenchymal stem cells. In some embodiments, the mesenchymal stem cells can be mesenchymal stem cells stem cells derived from bone marrow, adipose tissue, placental tissue, synovial tissue, umbilical cord tissue (for example, cord blood), dental pulp, or amniotic membrane, or mesenchymal stem cells differentiated from ES cells or iPS cells. In a preferred embodiment, the stem cells may be bone marrow-derived mesenchymal stem cells.

In still another aspect, the present disclosure provides cells obtained by the method and a pharmaceutical composition containing the cells. The cells and the pharmaceutical composition can be used for treating or preventing severe burns, spinal cord injury, head injury, cerebral infarction, cerebral hemorrhage, Parkinson's disease, or dementia.

### (Culture system, program, and the like)

In an aspect, the present disclosure provides a system for causing stem cells to proliferate while suppressing proliferation, the system including a culture vessel for culturing stem cells, and a temperature controller that controls a temperature of the culture vessel, in which the temperature controller controls the temperature to culture the stem cells under a hibernation condition of the stem cells.

In an aspect, the present disclosure provides a system for producing a predetermined amount of stem cells in a timely manner, the system including a culture vessel for culturing stem cells, a temperature controller that controls a temperature of the culture vessel, and a cell amount measuring means for detecting whether or not the amount of cells is a predetermined amount, in which the temperature controller controls the temperature to culture the stem cells under a hibernation condition and a normal culture condition of the stem cells.

In an aspect, the present disclosure provides a system for causing stem cells to proliferate while suppressing proliferation, the system including a culture vessel for culturing stem cells, and a temperature controller that controls a temperature of the culture vessel, in which the temperature controller controls the temperature to culture the stem cells at about 20°C to 34°C.

In an aspect, the present disclosure provides a system for producing a predetermined amount of stem cells in a timely manner, the system including a culture vessel for culturing stem cells, a temperature controller that controls a temperature of the culture vessel, and a cell amount measuring means for detecting whether or not the amount of cells is a predetermined amount, in which the temperature controller controls the temperature to culture the stem cells at about 20°C to 34°C and about 36°C to 38°C.

In some embodiments, the system of the present disclosure may be an automatic culture device further including a control unit that automatically controls a temperature control unit. The control unit can be controlled by a program or the like described in the present disclosure. Examples of the automatic culture device that can be used include, but are not limited to, Quantum (TERUMO BCT), CliniMACS Prodigy (Miltenyi Biotec), and CELLAFORTE (Nipro). Other automatic culture devices are disclosed in, for example, Miyuki SHIMIZU et al., Medical Instrumentation Vol. 88, No. 4 (2018) (17).

In the present disclosure, the culture vessel may be any vessel as long as stem cells can be cultured.

In the present disclosure, the temperature controller may be any device as long as the temperature of the culture vessel can be controlled. In addition to the mechanism for increasing or decreasing the normal temperature, a thermometer for measuring a temperature, a CPU storing a program for controlling a temperature, and the like are provided, but the present invention is not limited thereto.

The present disclosure also provides a program for controlling a culture device so that culture of stem cells can be performed according to the method of the present disclosure.

In an aspect, the present disclosure provides a program for causing a system for causing stem cells to proliferate while suppressing proliferation to execute a method for causing stem cells to proliferate while suppressing proliferation, the method including a step of culturing the stem cells under a hibernation condition of the stem cells.

In an aspect, the present disclosure provides a program for causing a system for causing stem cells to proliferate while suppressing proliferation to execute a method for causing stem cells to proliferate while suppressing proliferation, the method including a step of culturing the stem cells under a hibernation condition and a normal culture condition of the stem cells.

In an aspect, the present disclosure provides a program for causing a system for producing a predetermined amount of stem cells in a timely manner to execute a method for producing a predetermined amount of stem cells in a timely manner, the method including a step of culturing the stem cells under a hibernation condition and a normal culture condition of the stem cells.

In an aspect, the present disclosure provides a program for causing a system for producing a predetermined amount of stem cells in a timely manner to execute a method for producing a predetermined amount of stem cells in a timely manner, the method including a step of culturing the stem cells at about 20°C to 34°C and about 36°C to 38°C, and optionally, a step of confirming whether or not the amount of cells is a predetermined amount.

The present disclosure also provides a computer program for causing a computer to execute a method described in another part of the present disclosure or a storage medium storing the computer program, a system or user equipment constituting a part of the system, and the like.

Machine learning, neural network, and the like can be utilized to implement the method of the present disclosure. A weighting factor for each node of a hidden layer of the neural network may be calculated based on pre-obtained data. The processing of calculating the weighting factor is learning processing. The learning processing may be supervised learning or unsupervised learning. In the case of supervised learning, for example, the weighting factor of each node can be calculated so that a value of an output layer when a value of a certain parameter is input to an input layer becomes an appropriate amount or level. This processing can be performed, for example, by back propagation (error back propagation). Although it is preferable that the amount of training data set used for learning is large, when the amount is too large, over-learning is likely to occur. For implementation of the present disclosure, a learned model may be used, and the learned model is not limited to the neural network model as described, and any other machine learning model can be used. For example, a random forest may be used. For example, a simple model constructed by simply setting a weighting factor obtained by preliminary learning can also be used.

The program of the present disclosure can be operated on any terminal. The terminal may be, for example, any terminal device such as a smartphone, a tablet computer, a smart watch, a laptop computer, or a desktop computer. A user device may communicate with a server device via a network. Here, the type of the network is not limited. For example, the user device may communicate with the server device via the Internet or may communicate with the server device via a LAN. The number of user devices is limited thereto and may be any number of one or more. Furthermore, a database unit connected to the server device can store eyeball information of a plurality of targets acquired in advance. The stored information may be used, for example, to construct a learned model. For example, the constructed learned model may be stored in the database unit.

The user device includes a communication interface unit, an input unit, a display unit, a memory unit, and a processor unit.

The communication interface unit controls communication via a network. The processor unit of the user device can receive information from the outside of the user device via the communication interface unit, and can transmit information to the outside of the user device. For example, the processor unit of the user device can receive information from the server device via the communication interface unit, and can transmit information to the server device. The communication interface unit may control communication in any manner.

The input unit allows a user to input information to the user device. It does not matter in what manner the input unit enables the user to input information to the user device. For example, in a case where the input unit is a touch panel, the user may input information by touching the touch panel. Alternatively, in a case where the input unit is a mouse, the user may input information by operating the mouse. Alternatively, in a case where the input unit is a keyboard, the user may input information by pressing a key of the keyboard. Alternatively, in a case where the input unit is a microphone, the user may input information by voice.

The calculation result, the measurement, or the like of the present disclosure can be displayed on the display unit included in the system of the present disclosure. The display unit may be any display for displaying information.

A memory unit that can be included in the system of the present disclosure stores a program for executing processing in the user device, data required for executing the program, and the like. The memory unit stores, for example, a part or all of a program for implementing the system of the present disclosure and/or a program for analyzing or evaluating information related to a target. The memory unit may store an application that implements an arbitrary function.

The processor unit controls the operation of the entire user device. The processor unit reads a program stored in the memory unit and executes the program. As a result, it is possible to cause the user device to function as a device that executes a desired step. The processor unit may be implemented by a single processor or may be implemented by a plurality of processors.

The server device includes a communication interface unit, a memory unit, and a processor unit.

The communication interface unit controls communication via a network. In addition, the communication interface unit also controls communication with the database unit. The processor unit of the server device can receive information from the outside of the server device via the communication interface unit, and can transmit information to the outside of the server device. The processor unit of the server device can receive information from the user device via the communication interface unit, and can transmit information to the user device. The communication interface unit may control communication in any manner.

The memory unit stores a program required for execution of processing of the server device, data required for execution of the program, and the like.

The processor unit controls the operation of the entire server device. The processor unit reads a program stored in the memory unit and executes the program. As a result, it is possible to cause the server device to function as a device that executes a desired step. The processor unit may be implemented by a single processor or may be implemented by a plurality of processors.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples that are specifically described in the present specification, but is limited only by the claims.

The cells of the present disclosure may be cells used in cell infusion therapy and drug discovery research. In cell infusion therapy, the cells may be obtained from a subject or from a different subject.

In some embodiments, as a culture medium to be used, any culture medium may be used, for example, a platelet lysate is preferable. The platelet may be a platelet obtained from a subject or a platelet obtained from a different subject. The cells and platelets to be cultured may be of the same origin (that is, autologous) or of different origins (that is, xenogeneic). In a preferred embodiment, the platelet can be autologous.

Reference literatures such as scientific literatures, patents, and patent applications cited in the present specification are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples that are specifically described in the present specification, but is limited only by the claims.

### Examples

Hereinafter, the present disclosure will be described more specifically based on examples. The following examples were conducted in compliance with the Declaration of Helsinki and other ethical codes.

### (Example 1: Temperature change experiment 1 in cell culture)

In the present example, it was confirmed whether cell proliferation was suppressed by lowering the temperature for culturing from 37°C to 30°C at the time of cell culture.

### (Materials)

- Cells ... Human bone marrow-derived mesenchymal stem cells (BMSCs) (3 specimens)
- Medium ... MEM-α + 5% (platelet lysate) PL + (penicillin-streptomycin solution) PS
- Vessel ... FALCON EasyGrip Tissue Culture Dish 35 mm (bottom area 9 cm²)
- Incubator ... Set to 37°C and set to 30°C

### (Methods)

### -Day 0-

1. The cultured human BMSCs were exfoliated and recovered, and the number of cells was counted.
2. Thirteen 35 mm dishes were prepared, six dishes were designated as a group A, and six dishes were designated as a group B.
3. 2 ml of a medium was added to each dish.
4. 1.5 × 10⁴ cells were seeded in each dish.
5. The cells were cultured in an incubator at 37°C.

### -Day 2-

6. One dish was removed, the medium was removed, and washing was performed with PBS.
7. 1 ml of TrypLE was added, and a reaction was allowed to proceed at 37°C for 5 minutes.
8. 1 ml of a medium was added to inactivate TrypLE and collection into a Falcon tube was performed by pipetting.
9. Centrifugation was performed at 1,500 rpm for 5 minutes.
10. The cells were suspended in 1 ml of a medium and counted with LUNA-FL (trademark) (3 independent experiments).
11. The six dishes of the group A were transferred to an incubator at 30°C.

### -Day 3 to Day 5-

12. One dish was taken out from the group A, one dish was taken out from the group B, and the procedure of 6. to 10. on Day 2 was carried out.
13. On Day 5, the remaining dishes of the group A were returned to the incubator at 37°C.

### -Day 6 to Day 8-

14. The procedure of 12. on Day 3 to Day 5 was carried out.

### (Results)

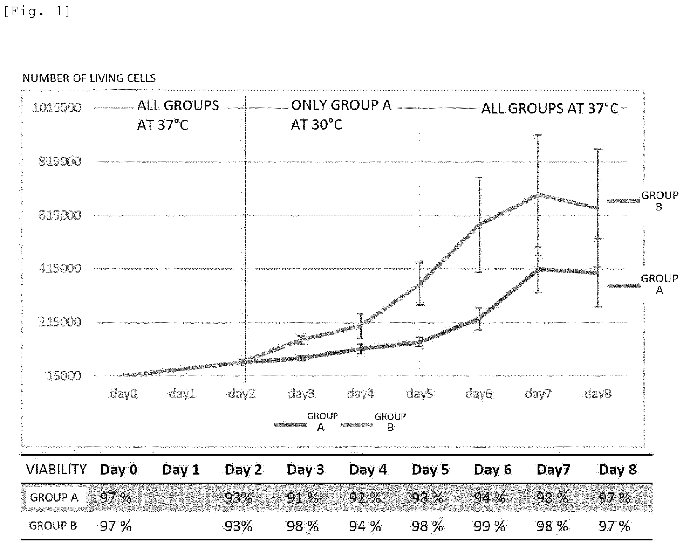

The results are shown in FIG. 1. As shown, a proliferation rate of stem cells decreased only during the low-temperature (30°C) period, and proliferation returned to normal for 4 days after rewarming (FIG. 1).

### (Example 2: Temperature change experiment 2 in cell culture)

In the present example, it was confirmed how cell proliferation was caused by lowering the temperature for culturing from 37°C to 30°C, 32°C, or 34°C during cell culture. Furthermore, it was confirmed what kind of influence was exerted on cell proliferation by rewarming from 30°C, 32°C, or 34°C to 37°C.

### (Materials)

- Cell ... Human BMSCs (3 specimens)
- Medium ... MEM-α + 5% PL + PS
- Vessel ... Nunc EasY Flask (bottom area 25 cm²)
- Incubator ... Set to 30°C, 32°C, 34°C, and 37°C

### (Methods)

### -Day 0-

1. BMSCs cryopreserved in liquid nitrogen were removed, thawed, and counted. Appropriate dilution was performed according to the number of cells.
2. Fifteen 25 cm² flasks were prepared for each temperature group.
3. 5 ml of a medium was added to each dish.
4. 1.0 × 10⁴ cells were seeded in each dish.
5. The cells were cultured in an incubator at 37°C.

### -Day 2-

6. One flask was removed, the medium was removed, and washing was performed with PBS.
7. 1 ml of TrypLE was added, and a reaction was allowed to proceed at 37°C for 5 minutes.
8. 1 ml of a medium was added to inactivate TrypLE and collection into a Falcon tube was performed by pipetting.
9. Centrifugation was performed at 1,500 rpm for 5 minutes.
10. The cells were suspended in 1 ml of a medium and counted with LUNA-FL (trademark).
11. The remaining four flasks in the group to which the temperature change was applied were transferred to an incubator at 30°C (32°C and 34°C).

### -Day 3 to Day 5-

12. One flask was removed and the procedure from 6. to 10. on Day 2 was carried out.
13. On Day 5, the remaining dishes of the group A were returned to the incubator at 37°C.

### -Day 6 to Day 8-

14. The procedure of 12. on Day 3 to Day 5 was carried out.

### (Results)

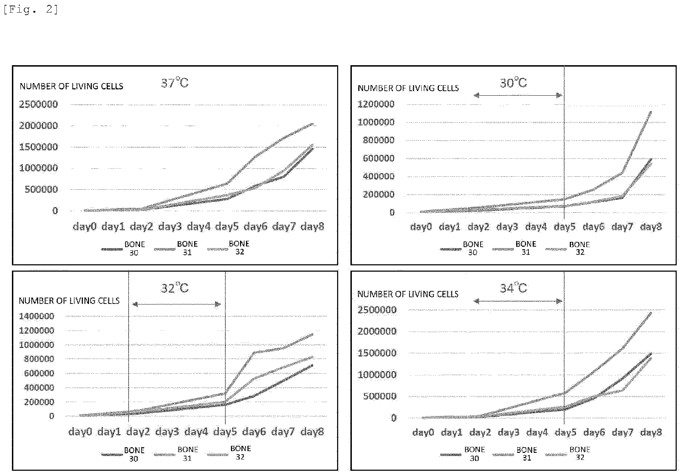

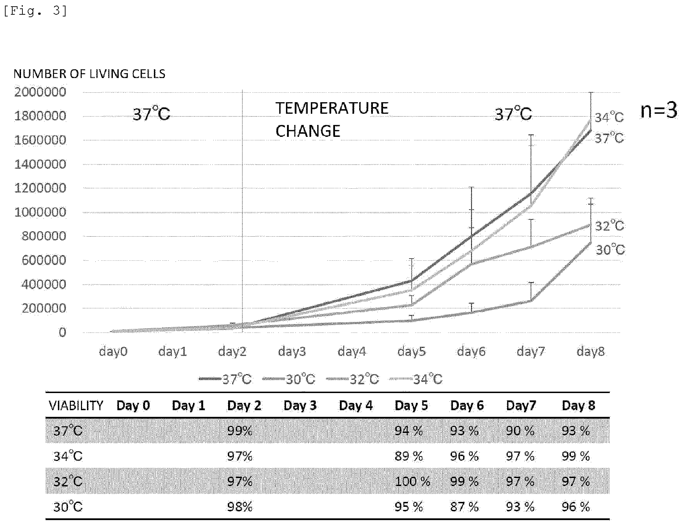

The results are shown in FIGS. 2 and 3. As shown, cell proliferation was suppressed at 30°C and 32°C. Cell proliferation was slightly suppressed even at 34°C. In addition, after rewarming to 37°C, the cells proliferated normally even when cultured at any temperature change.

### (Example 3: Temperature change experiment 3 in long-term cultured cells)

In the present example, the culture period was extended to a total of 14 days so that confluence was observed, and the influence of the temperature change was confirmed. However, when confluence was confirmed during the culture, the process was ended at that time. The three-day period of applying the temperature change was not changed.

### (Methods)

- Human BMSCs (3 specimens)
- Medium (MEM-α+5% PL + P/S)
- Nunc EasY Flask25cm²
- Incubator (37°C and 32°C)

### (Methods)

### -Day 0-

1. BMSCs cryopreserved in liquid nitrogen were removed, thawed, and counted. Appropriate dilution was performed according to the number of cells.
2. Twenty four (12 × 2) 25 cm² flasks were prepared for each temperature condition.
3. 5 ml of a medium was added to each dish.
4. 1.0 × 10⁴ cells were seeded in each dish.
5. The cells were cultured in an incubator at 37°C.

### -Day 2-

6. One flask was removed, the medium was removed, and washing was performed with PBS.
7. 1 ml of TrypLE was added, and a reaction was allowed to proceed at 37°C for 5 minutes.
8. 1 ml of a medium was added to inactivate TrypLE and collection into a Falcon tube was performed by pipetting.
9. Centrifugation was performed at 1,500 rpm for 5 minutes.
10. The cells were suspended in 1 ml of a medium and counted with LUNA-FL (trademark).
11. The remaining flasks in the group to which the temperature change was applied were transferred to an incubator at 32°C.

### -Day 5-

12. One flask was taken out from 32°C, one dish was taken out from 37°C, and the procedure of 6. to 10. on Day 2 was carried out.
13. The flask that was transferred to an incubator at 32°C was returned to the incubator at 37°C.

### -Day 6 to Day 14-

14. Counting from the group to which the temperature change of 32°C was added and the group that remained at 37°C was performed on one flask per day.
15. When confluence was confirmed by microscopic observation, counting was not performed from the next day.

### (Results)

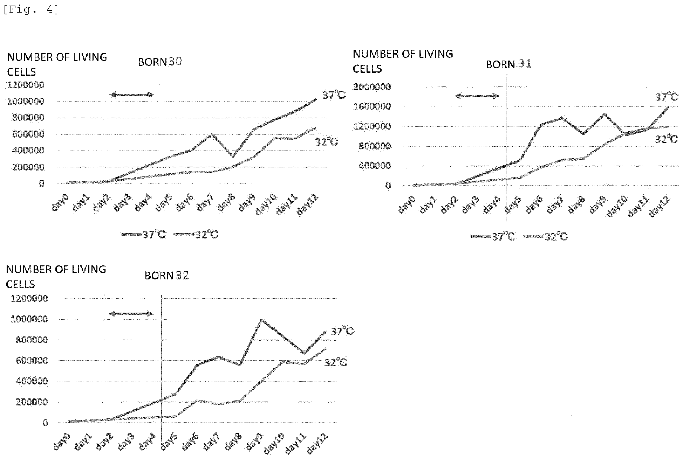

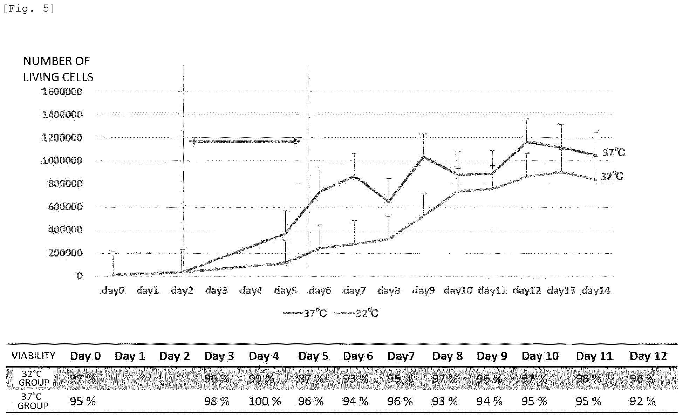

As in Examples 1 and 2, the proliferation rate of stem cells decreased only during the low-temperature period, and after rewarming, proliferation returned to normal (FIGS. 4 and 5).

### (Example 4: Change in trophic factors according to temperature change)

It was confirmed whether a change in secretion of trophic factors of cells was observed by applying a temperature change and the time of applying the temperature change.

### (Materials)

- Cell ... Human BMSCs (2 specimens)
- Medium ... MEM-α + 5% PL + PS
- Vessel ... Nunc EasY Flask 25 cm²
- Incubator ... set to 37°C and 32°C
- ELISA kit ... HGF (trophic factors not included in PL)

### (Methods)

Human BMSCs were cultured at 37°C for 2 days, and shifted to low-temperature culture (for 0, 1, 3, 5, and 7 days). Thereafter, rewarming culture was performed at 37°C for 3 days, and then trophic factors were measured. 1,000 µL of a supernatant was collected, a concentration of the trophic factors was determined by the ELISA method, the number of cells was counted, and the amount of the trophic factors per cell was calculated.

Experiments were performed on two samples for each of the groups: no temperature change group, 1 day temperature change group, 3 day temperature change group, 5 day temperature change group, and 7 day temperature change group (10 samples in total).

### (Results)

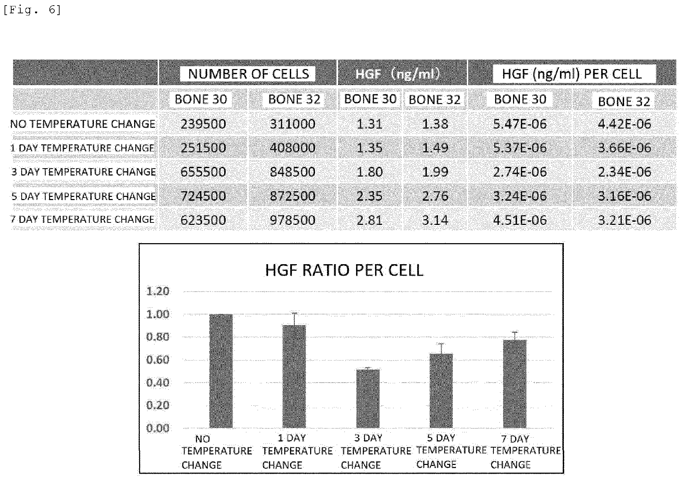

The results are shown in FIG. 6. It is considered that there is no large difference in trophic factor secretion among the temperature change groups. Although the amount of HGF appears to be low in the 3 day temperature change group, it is considered to be due to the fact that cell proliferation is unexpectedly fast, the number of cells is large, and the denominator is large.

### (Example 5: Examination of automatic culture device)

In the present example, the influence of temperature change in cell culture using an automatic culture device was confirmed.

### (Materials)

- Cell ... Human PBMCs
- Culture device ... Quantum automatic culture device
- Medium ... MEM-α + 5% PL + PS

### (Methods)

The cells were cultured at 37°C until the number of cells reached 10 to 50 million (4 to 11 days), and then transferred to a low temperature of 28 to 30°C and cultured (4 to 10 days). Thereafter, the cells were rewarmed to 37°C and cultured for 4 to 6 days. Since a value other than the final number of cells could not be measured, the lactic acid value was converted into the number of cells (4.34e-8 mmol/day/cell).

### (Results)

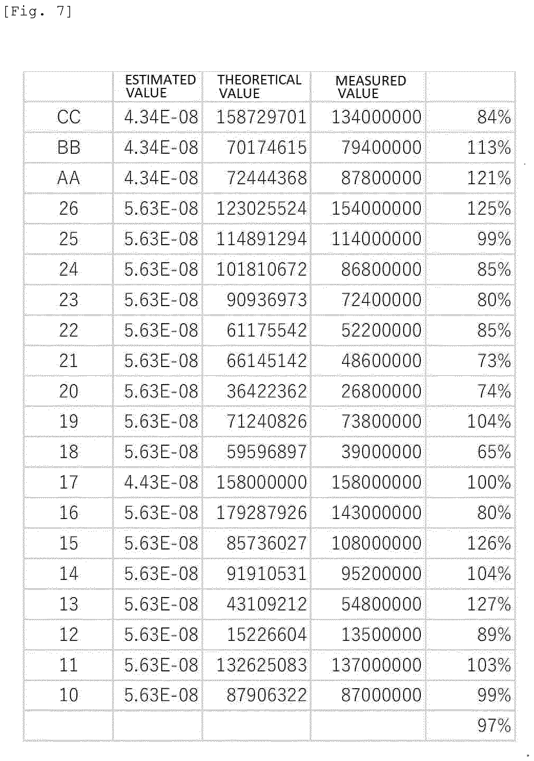

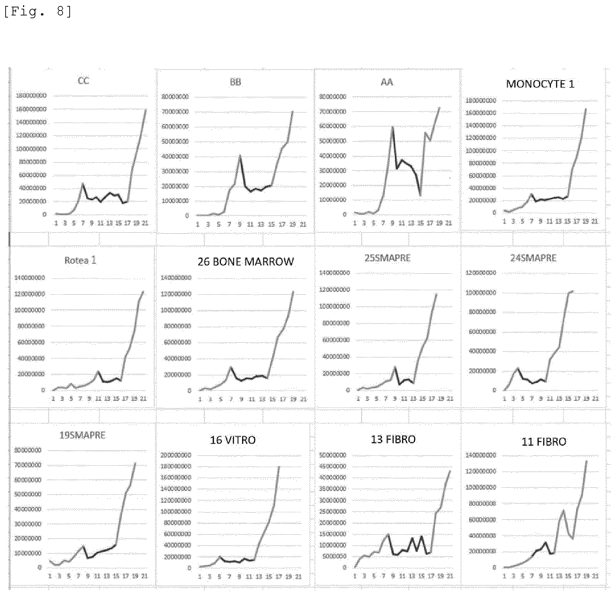

When the final number of cells was compared with the number of cells converted from the lactic acid value, the number of cells converted from the lactic acid value matched the measured value with an accuracy of 97 ± 19% (FIG. 7). Also in the automatic culture device, cell proliferation was suppressed in the low-temperature period, and cell proliferation was observed without any problem after rewarming (FIG. 8).

### (Example 6: Temperature change experiment 1 in cell culture (20°C))

In the present example, it was confirmed what kind of influence was exerted on cell proliferation by the temperature change to a temperature lower than those in Examples 1 to 5 (20°C).

### (Materials)

- Cell ... Human BMSCs
- Medium ... MEM-α + 5% PL + PS
- Vessel ... FALCON EasyGrip Tissue Culture Dish 35 mm (bottom area 9 cm²)
- Incubator ... A normal cell culture incubator was used at 37°C. At 20°C, a 5% CO² environment was created using Anaeropack in AS ONE ICI100.

### (Methods)

### -Day 0-

1. The human BMSCs were exfoliated and recovered during culture, and the number of cells was counted.
2. Thirteen 35 mm dishes were prepared. Six dishes were designated as a group A and six dishes were designated as a group B.
3. 2 ml of a medium was added to each dish.
4. 3.6 × 10³ cells were seeded in each dish.
5. The cells were cultured in an incubator at 37°C.

### -Day 2-

6. One dish was removed, the medium was removed, and washing was performed with PBS.
7. 1 ml of TrypLE was added, and a reaction was allowed to proceed at 37°C for 5 minutes.
8. 1 ml of a medium was added to inactivate TrypLE and collection into a Falcon tube was performed by pipetting.
9. Centrifugation was performed at 1,500 rpm for 5 minutes.
10. The cells were suspended in 1 ml of a medium and counted with LUNA-FL (trademark).
11. The six dishes of the group A were put in an AnaeroPack jar, an AnaeroPack was put therein, and a lid was immediately closed.
12. 11. was placed in AS ONE ICI100 set to 20°C.

### -Day 3 to Day 5-

13. An AnaeroPack jar was opened, one dish was taken out from the group A, one dish was taken out from the group B from the incubator at 37°C, and the procedure of 6. to 10. on Day 2 was carried out.
14. A new AnaeroPack was opened, replacement with an AnaeroPack jar was performed, and the temperature was returned to 20°C.
15. On Day 5, the remaining dishes of the group A were all transferred to an incubator at 37°C.

### -Day 6 to Day 8-

16. One dish was taken out from the group A, one dish was taken out from the group B, and the procedure of 6. to 10. on Day 2 was carried out.

### (Results)

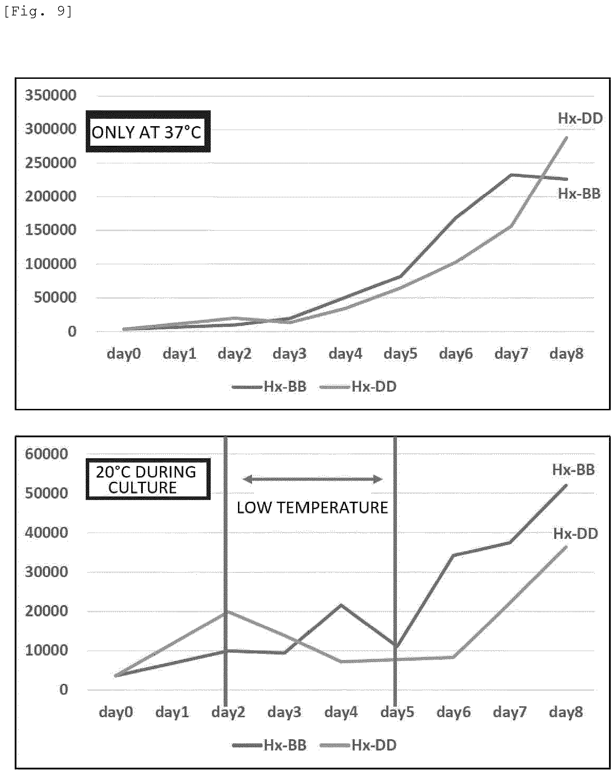

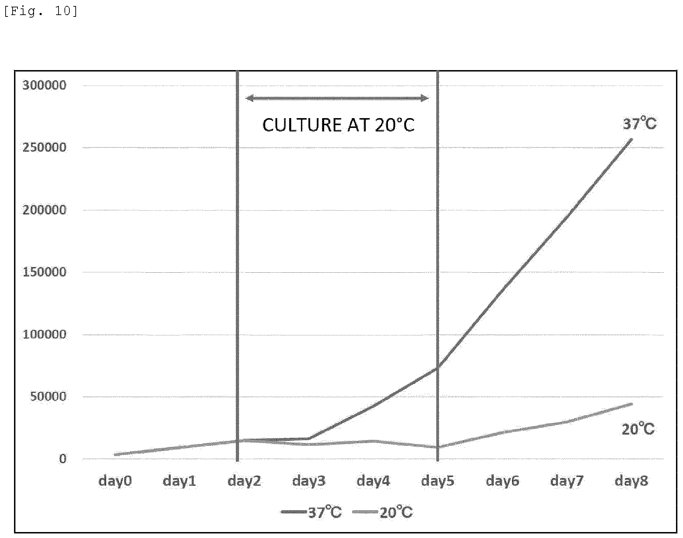

In the culture at 20°C, cell proliferation was suppressed, and cell proliferation returned after rewarming to 37°C, but the proliferation rate was relatively slow (FIGS. 9 and 10).

### (Example 7: Temperature change experiment 1 in cell culture (25°C))

In the present example, it was confirmed what kind of influence was exerted on cell proliferation by the temperature change to a temperature lower than those in Examples 1 to 5 (25°C).

### (Materials)

- Cell ... Human BMSCs
- Medium ... MEM-α + 5% PL + PS
- Vessel ... FALCON EasyGrip Tissue Culture Dish 35 mm (bottom area 9 cm²)
- Incubator ... set to 37°C

At 25°C, a 5% CO² environment was created using Anaeropack in AS ONE ICI100.

### (Methods)

### -Day 0-

1. The human BMSCs were exfoliated and recovered during culture, and the number of cells was counted.
2. Thirteen 35 mm dishes were prepared. Six dishes were designated as a group A and six dishes were designated as a group B.
3. 2 ml of a medium was added to each dish.
4. 7.2 × 10³ cells were seeded in each dish.
5. The cells were cultured in an incubator at 37°C.

### -Day 2-

6. One dish was removed, the medium was removed, and washing was performed with PBS.
7. 1 ml of TrypLE was added, and a reaction was allowed to proceed at 37°C for 5 minutes.
8. 1 ml of a medium was added to inactivate TrypLE and collection into a Falcon tube was performed by pipetting.
9. Centrifugation was performed at 1,500 rpm for 5 minutes.
10. The cells were suspended in 1 ml of a medium and counted with LUNA-FL (trademark).
11. The six dishes of the group A were put in an AnaeroPack jar, an AnaeroPack was put therein, and a lid was immediately closed.
12. 11. was placed in AS ONE ICI100 set to 25°C.

### -Day 3 to Day 5-

13. An AnaeroPack jar was opened, one dish was taken out from the group A, one dish was taken out from the group B from the incubator at 37°C, and the procedure of 6. to 10. on Day 2 was carried out.
14. A new AnaeroPack was opened, replacement with an AnaeroPack jar was performed, and the temperature was returned to 25°C.
15. On Day 5, the remaining dishes of the group A were all transferred to an incubator at 37°C.

### -Day 6 to Day 8-

16. One dish was taken out from the group A, one dish was taken out from the group B, and the procedure of 6. to 10. on Day 2 was carried out.

### (Results)

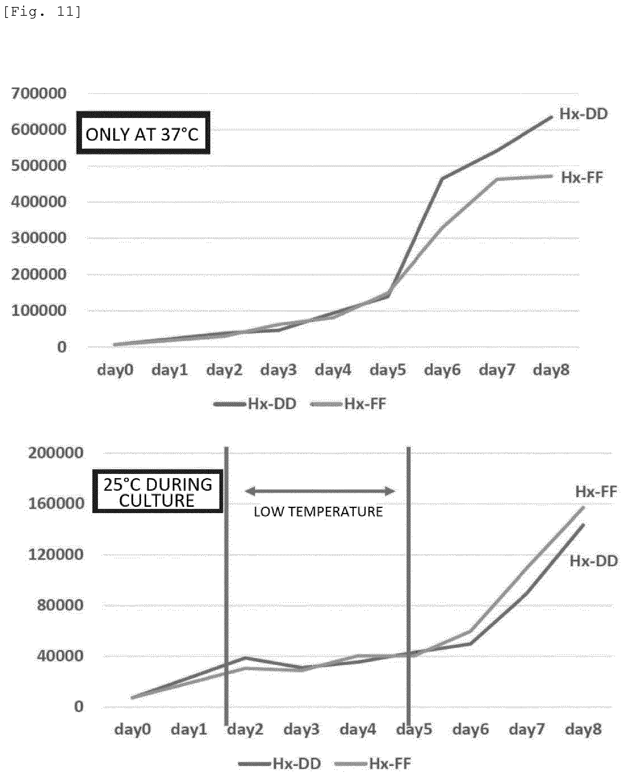

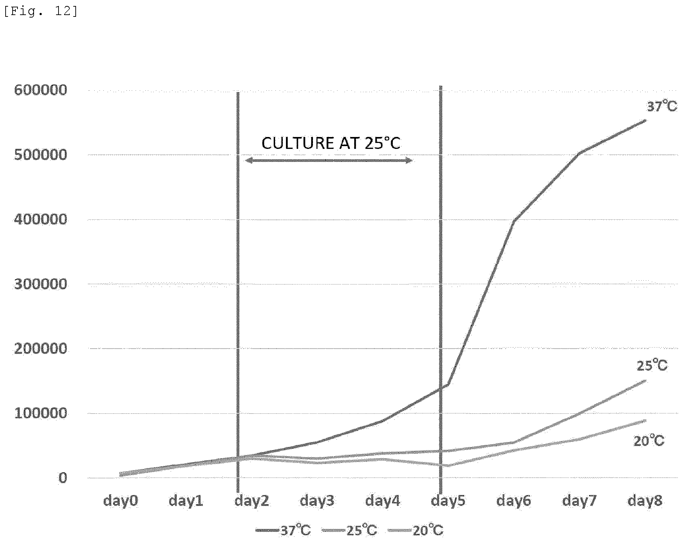

When the temperature was set to 25°C, cell proliferation was suppressed, and cells normally proliferated after rewarming. The proliferation rate after rewarming was higher than that in culture at 20°C in Example 6 (FIGS. 11 and 12).

### (Discussion)

From the above, cell proliferation of stem cells (for example, bone marrow-derived mesenchymal stem cells) was suppressed at 20°C to 34°C, and the hibernation condition of stem cells was clarified. In addition, it is important that cells proliferate normally after rewarming. However, cell proliferation returned although there was a difference in proliferation rate under any hibernation condition. Although it is possible to appropriately set the temperature of the hibernation condition according to the desired number of cells, since the rate of cell proliferation after rewarming is slow at 20°C and there is a little difference at 34°C in the final number of cells from the case in which the cells are cultured constantly at 37°C, it is preferable to use a hibernation condition of 25°C to 32°C, and it is more preferable to use a hibernation condition of 30°C to 32°C. The proliferation rate of bone marrow-derived mesenchymal stem cells greatly varies depending on the donor, the nationality of the subject, and the facility in which the cells are cultured (Stroncek1 et al., Front Cell Dev Biol. 2020 Jun 16; 8:458. doi: 10.3389/fcell.2020.00458. eCollection 2020), but according to the method of the present disclosure, a target final number of cells can be achieved by appropriately controlling the temperature and period during culture.

### (Example 8) Example protocol for actually producing appropriate amount

50 ml of a bone marrow fluid in which the number of contained stem cells is unknown is put into an automatic culture device, and the adherent stem cells are cultured up to 100 million in 4 weeks. The degree of proliferation during culture is estimated from a daily secretion amount of a known lactic acid value that is output by each stem cell.

### (Example 9) Treatment example after actually producing appropriate amount of cell preparation

For local damage of the brain (cerebral infarction, intracerebral hemorrhage, or traumatic brain injury) and the like, rehabilitation can be obtained by administering 40 million cells around the damaged site.

As described above, the present disclosure is exemplified by the use of its preferred embodiments. It is understood that the scope of the present disclosure should be interpreted solely based on the claims. It is also understood that any patent, any patent application, and any references cited herein should be incorporated herein by reference in the same manner as the contents are specifically described herein. The present application claims priority to Japanese Patent Application No. 2023-011235 filed on January 27, 2023 to the Japan Patent Office, the entire contents of which are incorporated herein by reference.

### Industrial Applicability

According to the present disclosure, it is possible to control a proliferation rate of stem cells, and it is possible to produce an appropriate amount of stem cells in a timely manner. Since the cultured cells are used for cell infusion therapy, the cultured cells can be used in the fields of pharmaceuticals and the like.

## Claims

1. A method for causing stem cells to proliferate while suppressing proliferation, the method comprising a step of culturing the stem cells under a hibernation condition of the stem cells.

2. A method for causing stem cells to proliferate while suppressing proliferation, the method comprising a step of culturing the stem cells at a temperature of about 20°C to about 34°C.

3. The method according to claim 1, wherein the hibernation condition is a temperature of about 25°C to about 34°C.

4. The method according to claim 1, wherein the hibernation condition is a temperature of about 28°C to about 32°C.

5. The method according to claim 1, wherein the hibernation condition is a temperature of about 30°C to about 32°C.

6. The method according to any one of claims 1 to 5, wherein the method is performed without passaging.

7. The method according to any one of claims 1 to 6, wherein the method is performed in an automatic culture device.

8. The method according to any one of claims 1 to 7, wherein the stem cells are mesenchymal stem cells.

9. The method according to claim 8, wherein the mesenchymal stem cells are mesenchymal stem cells stem cells derived from bone marrow, adipose tissue, placental tissue, synovial tissue, umbilical cord tissue (for example, cord blood), dental pulp, or amniotic membrane, or mesenchymal stem cells differentiated from ES cells or iPS cells.

10. The method according to claim 9, wherein the mesenchymal stem cells are bone marrow-derived mesenchymal stem cells.

11. The method according to any one of claims 1 to 10, wherein the cells are cells used in any of cell infusion therapy and drug discovery research.

12. A method for producing a predetermined amount of stem cells in a timely manner, the method comprising:
a step (a) of culturing the stem cells under a hibernation condition of the stem cells; and
a step (b) of culturing the stem cells to a predetermined amount of the stem cells at about 36°C to 38°C.

13. The method according to claim 12, wherein the hibernation condition is a temperature of about 20°C and to 34°C.

14. The method according to claim 12, wherein the hibernation condition is from about 28°C to about 32°C.

15. The method according to claim 12, wherein the hibernation condition is from about 30°C to about 32°C.

16. A method for producing a predetermined amount of stem cells in a timely manner, the method comprising:
a step (a) of culturing the stem cells at a temperature of the stem cells of about 20°C to about 34°C; and
a step (b) of culturing the stem cells to a predetermined amount of the stem cells at about 36°C to 38°C.

17. The method according to any one of claims 12 to 16, wherein the method is performed without passaging.

18. The method according to any one of claims 12 to 17, wherein the method is performed in an automatic culture device.

19. The method according to any one of claims 12 to 18, wherein the stem cells are mesenchymal stem cells.

20. The method according to claim 19, wherein the mesenchymal stem cells are mesenchymal stem cells stem cells derived from bone marrow, adipose tissue, placental tissue, synovial tissue, umbilical cord tissue (for example, cord blood), dental pulp, or amniotic membrane, or mesenchymal stem cells differentiated from ES cells or iPS cells.

21. The method according to claim 19, wherein the mesenchymal stem cells are bone marrow-derived mesenchymal stem cells.

22. The method according to any one of claims 12 to 21, wherein the cells are cells used in cell infusion therapy and drug discovery research.

23. Cells obtained by the method according to any one of claims 1 to 22.

24. A pharmaceutical composition comprising cells obtained by the method according to any one of claims 1 to 22.

25. The pharmaceutical composition according to claim 24, wherein the pharmaceutical composition is used for treating or preventing severe burns, spinal cord injury, head injury, cerebral infarction, cerebral hemorrhage, Parkinson's disease, or dementia.

26. A system for causing stem cells to proliferate while suppressing proliferation, the system comprising:
a culture vessel for culturing stem cells; and
a temperature controller that controls a temperature of the culture vessel,
wherein the temperature controller controls the temperature to culture the stem cells under a hibernation condition of the stem cells.

27. A system for producing a predetermined amount of stem cells in a timely manner, the system comprising:
a culture vessel for culturing stem cells;
a temperature controller that controls a temperature of the culture vessel; and
a cell amount measuring means for detecting whether or not the amount of cells is a predetermined amount,
wherein the temperature controller controls the temperature to culture the stem cells under a hibernation condition and a normal culture condition of the stem cells.

28. A system for causing stem cells to proliferate while suppressing proliferation, the system comprising:
a culture vessel for culturing stem cells; and
a temperature controller that controls a temperature of the culture vessel,
wherein the temperature controller controls the temperature to culture the stem cells at about 20°C to 34°C.

29. A system for producing a predetermined amount of stem cells in a timely manner, the system comprising:
a culture vessel for culturing stem cells;
a temperature controller that controls a temperature of the culture vessel; and
a cell amount measuring means for detecting whether or not the amount of cells is a predetermined amount,
wherein the temperature controller controls the temperature to culture the stem cells at about 20°C to 34°C and about 36°C to 38°C.
